# EUROPEAN PATENT SPECIFICATION

(11) **EP 2 822 585 B1**
(45) Date of publication and mention of the grant of the patent: **17.05.2017**
(21) Application number: 13707395.3
(22) Date of filing: 05.03.2013
(51) Int. Cl.: A61K 39/145

(54) **IMPROVED VACCINATION AGAINST INFLUENZA**
VERBESSERTER IMPFSTOFF GEGEN GRIPPE
VACCINATION AMÉLIORÉE CONTRE LA GRIPPE

(30) Priority: 06.03.2012 US 201261607439 P; 06.03.2012 EP 12158258; 02.04.2012 US 201261619293 P; 05.10.2012 US 201261710404 P
(43) Date of publication of application: 14.01.2015
(73) Proprietor: Janssen Vaccines & Prevention B.V., 2333 CN Leiden (NL)
(72) Inventor: ROOZENDAAL, Ramon, 2333 CN Leiden (NL); ROOS, Anna, 2333 CN Leiden (NL); RADOSEVIC, Katarina, 2333 CN Leiden (NL)
(74) Representative: Manten, Annemieke
(86) International application number: PCT/EP2013/054378
(87) International publication number: WO 2013/131898

(56) References cited:
- WO-A1-2008/037033
- WO-A1-2008/128939
- WO-A1-2009/071633
- WO-A1-2010/092476
- WO-A1-2010/114169
- ZHIWEI SUI ET AL: "Cross-protection against influenza virus infection by intranasal administration of M2-based vaccine with chitosan as an adjuvant", ARCHIVES OF VIROLOGY ; OFFICIAL JOURNAL OF THE VIROLOGY DIVISIONOF THE INTERNATIONAL UNION OF MICROBIOLOGICAL SOCIETIES, SPRINGER-VERLAG, VI, vol. 155, no. 4, 27 February 2010 (2010-02-27), pages 535-544, XP019805216, ISSN: 1432-8798
- LEROUX-ROELS ET AL: "Antigen sparing and cross-reactive immunity with an adjuvanted rH5N1 prototype pandemic influenza vaccine: a randomised controlled trial", THE LANCET, LANCET LIMITED. LONDON, GB, vol. 370, no. 9587, 16 August 2007 (2007-08-16), pages 580-589, XP022202258, ISSN: 0140-6736, DOI: 10.1016/S0140-6736(07)61297-5
- KENNETH M. ZANGWILL ET AL: "Evaluation of the Safety and Immunogenicity of a Booster (Third) Dose of Inactivated Subvirion H5N1 Influenza Vaccine in Humans", THE JOURNAL OF INFECTIOUS DISEASES, vol. 197, no. 4, 15 February 2008 (2008-02-15), pages 580-583, XP055062175, ISSN: 0022-1899, DOI: 10.1086/526537
- VAN MAURIK A ET AL: "Seasonal influenza vaccine elicits heterosubtypic immunity against H5N1 that can be further boosted by H5N1 vaccination", VACCINE, ELSEVIER LTD, GB, vol. 28, no. 7, 17 February 2010 (2010-02-17), pages 1778-1785, XP026884512, ISSN: 0264-410X, DOI: 10.1016/J.VACCINE.2009.12.008 [retrieved on 2009-12-16] cited in the application
- ICHINOHE ET AL.: "Cross-protection against H5N1 influenza virus infection is afforded by intranasal inoculation with seasonal trivalent inactivated influenza vaccine", J.INF.DISEASE, vol. 196, 2007, pages 1313-1320, XP009135118, cited in the application
- S. HAUGE ET AL: "Quality and Kinetics of the Antibody Response in Mice after Three Different Low-Dose Influenza Virus Vaccination Strategies", CLINICAL AND VACCINE IMMUNOLOGY, vol. 14, no. 8, 1 August 2007 (2007-08-01) , pages 978-983, XP055062012, ISSN: 1556-6811, DOI: 10.1128/CVI.00033-07
- HERZOG C ET AL: "Eleven years of Inflexal<(>R) V-a virosomal adjuvanted influenza vaccine", VACCINE, ELSEVIER LTD, GB, vol. 27, no. 33, 16 July 2009 (2009-07-16) , pages 4381-4387, XP026238159, ISSN: 0264-410X, DOI: 10.1016/J.VACCINE.2009.05.029 [retrieved on 2009-05-29]
- DELORE V ET AL: "Long-term clinical trial safety experience with the inactivated split influenza vaccine, Vaxigrip<(>R)", VACCINE, ELSEVIER LTD, GB, vol. 24, no. 10, 6 March 2006 (2006-03-06) , pages 1586-1592, XP028010507, ISSN: 0264-410X, DOI: 10.1016/J.VACCINE.2005.10.008 [retrieved on 2006-03-06]
- HOUSER KATHERINE V ET AL: "Seasonal Trivalent Inactivated Influenza Vaccine Does Not Protect against Newly Emerging Variants of Influenza A (H3N2v) Virus in Ferrets", JOURNAL OF VIROLOGY, vol. 87, no. 2, January 2013 (2013-01), pages 1261-1263, XP9169436,
- M. B. PEARCE ET AL: "Seasonal Trivalent Inactivated Influenza Vaccine Protects against 1918 Spanish Influenza Virus Infection in Ferrets", JOURNAL OF VIROLOGY, vol. 86, no. 13, 1 July 2012 (2012-07-01), pages 7118-7125, XP055062019, ISSN: 0022-538X, DOI: 10.1128/JVI.00674-12

## Description

The invention relates to the field of health care. More particularly, it concerns improved vaccination schemes to establish broad protection against influenza.

### Background of the invention

Influenza viruses are major human and animal pathogens, causing a respiratory disease, influenza, that may range in severity from sub-clinical infection to primary viral pneumonia which can result in death. The virus infects up to 1 billion people world-wide every year.

Many people get vaccination against influenza, by so-called seasonal influenza vaccine which preferably is administered once a year. The composition of the seasonal influenza vaccine typically changes every year.

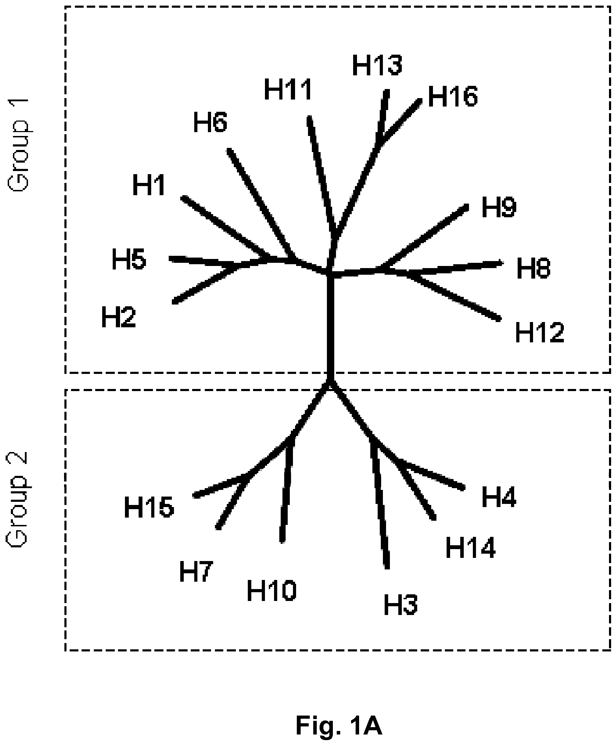

Influenza viruses are classified on the basis of differences in antigenic structure of their hemagglutinin (HA) and neuraminidase (NA) proteins, with their different combinations representing unique virus subtypes that are further classified into specific influenza virus strains. Although all known influenza A subtypes can be found in birds, currently circulating human influenza A subtypes are H1N1 and H3N2. Phylogenetic analysis has demonstrated a subdivision of hemagglutinins into two main groups: inter alia the H1, H2, H5 and H9 subtypes in phylogenetic group 1 and inter alia the H3, H4 and H7 subtypes in phylogenetic group 2 (see Fig. 1A). A seasonal influenza vaccine at this moment typically comprises HA and NA influenza proteins from strains of influenza A phylogenetic groups 1 (e.g. H1N1) and 2 (e.g. H3N2) and from influenza B.

It is known that antibodies that neutralize the influenza virus are primarily directed against HA. HA is a trimeric glycoprotein that is anchored to the viral coat and comprises a large head domain and a smaller stem domain.

The reason that the seasonal influenza vaccine must be updated every year is the large variability of the virus. In the HA molecule this variation is particularly manifested in the head domain where antigenic drift has resulted in a large number of different variants. Since this is also the area that is immunodominant, most neutralizing antibodies are directed against this domain.

The combination of immunodominance and large variation of the head domain also explains why infection with a particular strain does not lead to immunity to other strains: the antibodies elicited by the first infection only recognize a limited number of strains closely related to the virus of the primary infection.

The current immunization practice relies on early identification of circulating influenza viruses to allow for timely production of an effective seasonal influenza vaccine. This practice limits the time window for production and standardization of the vaccine to a maximum of nine months, which amounts to a yearly recurring challenging task from logistic and manufacturing perspective. A further disadvantage is that the chosen vaccine may not cover all circulating strains, resulting in a vaccine that is suboptimal in protection against seasonal influenza strains. Apart from the inherent difficulties in predicting the strains that will be dominant during the next season, antiviral resistance and immune escape also play a role in failure of current vaccines to prevent morbidity and mortality. In addition to this the possibility of a pandemic caused by a highly virulent strain originating from animal reservoirs and reassorted to increase human to human spread, poses a significant and realistic threat to global health. Even yearly standard use of seasonal vaccines does not provide protection against potentially newly emerging pandemic strains, such as H5N1 strains.

Increase in the breadth of protection by influenza vaccines has been observed in some studies, but required strong adjuvants, or mucosal (intranasal) administration, or in several cases both adjuvant and mucosal administration, or whole viruses of very specific highly immunogenic influenza strains (Tumpey et al., 2001, J.Virology, 75: 5141-5150; Ichinohe et al., 2007, J.Inf.Disease 196: 1313-1320; van Maurik et al., 2010, Vaccine 28: 1778-1785; WO 2008/037033; Tamura et al, 1989, Vaccine 7: 314-320; Zhiwei Sui et al, 2010, Archives of Virol 155: 535-544). In most of those cases, the cross-protection reported was still not broad. A disadvantage of mucosal administration is that immunological memory is poorly induced. Disadvantages of most or all of the adjuvants used in those reports are that these are not recommended for human use, due to reactogenicity.

Thus, there remains a need in the art for improved influenza vaccination. The present invention aims at providing improved vaccination for influenza, in particular to improve the breadth of protection of influenza vaccines.

### Summary of the invention

Contrary to the established dogma of strain-specific immunity induction by influenza vaccines, it was surprisingly discovered by the present inventors that vaccinating with an influenza vaccine that comprises HA and NA proteins induces broad protection against heterologous and even heterosubtypic influenza strains, as compared to the strains from which the influenza proteins in the vaccine were derived, if the vaccine was administered three times, even without added adjuvant, and administration via an injection route.

The invention also provides a vaccine comprising HA and NA proteins from at least a first influenza strain, for use as defined in the claims.

The vaccine used according to the invention is a seasonal influenza vaccine, comprising HA and NA from at least three influenza virus strains, and is a virosomal influenza vaccine, does not comprise an adjuvant and is administered intramuscularly.

The invention also provides a vaccine for use in inducing cross-protection against different influenza as defined in the claims.

The invention also provides an influenza vaccine comprising HA and NA, for use in inducing cross-protection against at least one heterosubtypic influenza strain as compared to the influenza strains from which antigens are present in the influenza vaccine, by administering the vaccine to a subject at least three times within one year.

In another aspect, the invention provides a method for inducing cross-protection against a H5N1 influenza strain, for the use as defined in the claims. The invention further provides an influenza vaccine that comprises HA and NA from an H1N1, an H3N2 and a B strain of influenza, for use in inducing cross-protection against a H5N1 influenza strain by administering the influenza vaccine to a subject at least three times within one year, wherein no HA and NA from the H5N1 influenza strain is administered to the subject.

The invention also provides an influenza vaccine that comprises HA and NA from an H1N1, an H3N2 and a B strain of influenza, for use in inducing cross-protection against a pandemic or potentially pandemic influenza strain, by administering the influenza vaccine to a subject at least three times within one year, wherein no HA and NA from the pandemic influenza strain is administered to the subject.

The invention also provides a seasonal vaccine comprising HA and NA proteins of at least three influenza strains, for use in inducing protection against the three influenza strains and cross-protection against at least one heterologous influenza strain within the same subtype as compared to at least one of the influenza strains, and also against at least one heterosubtypic influenza strain of which no HA and NA antigens are present in the vaccine, and wherein the vaccine is a virosomal influenza vaccine, and wherein the vaccine does not comprise an adjuvant and is administered intramuscularly to a subject at least three times within one year.

An influenza vaccine used according to the present invention thus does not comprise HA and NA from all strains to which it confers protection. Typically it also confers protection to at least one heterosubtypic influenza strain as compared to all strains from which HA and NA are represented in the vaccine, upon three times (i.e. prime and two boosts) administration of the vaccine within one year.

The influenza vaccine used in the invention is a regular and/or seasonal influenza vaccine. According to the invention, it is a virosomal influenza vaccine.

In certain embodiments, the influenza vaccine comprises HA and NA from three influenza strains (trivalent). In other embodiments, the influenza vaccine comprises HA and NA from four influenza strains (quadrivalent). In yet further embodiments, the influenza vaccine comprises HA and NA from at least five influenza strains (pentavalent and more). In certain embodiments the HA and NA in the vaccine comprise HA and NA from an influenza A strain. In certain embodiments, the HA and NA in the vaccine comprise HA and NA from an influenza H1N1 strain. In certain embodiments, the HA and NA in the vaccine are from an influenza H1 strain, an influenza H3 strain and an influenza B strain. In certain embodiments, the HA and NA in the vaccine are from a H1N1 strain, a H3N2 strain and a B strain.

In certain embodiments, the vaccine comprises HA and NA from a first influenza H1 strain and induces protection against said first strain and against at least a second influenza H1 strain that is heterologous to said first strain.

In certain embodiments, the vaccine induces protection against at least one strain that is heterosubtypic with respect to all influenza strains represented in the vaccine.

In certain embodiments, the vaccine comprises HA and NA from a first influenza H1 strain and induces protection against said first strain and against at least a second influenza strain which is an influenza H5 strain, wherein the vaccine does not comprise HA and NA from an influenza H5 strain.

In certain embodiments, the vaccine is administered to the subject at least three times within 11, 10, 9, 8, 7, 6, 5, 4, 3 months or less. In certain embodiments, the administration of the vaccine is performed with an interval between two administrations of at least one week, two weeks, three weeks, or four weeks. In certain embodiments, the administration of the vaccine is performed with an interval between two administrations of between about three to eight weeks. In certain embodiments, the vaccine is administered to a subject with an interval between two administrations of not more than six months, five months, four months, or three months. In certain embodiments, the second dose of the vaccine is administered to the subject at between 1 week and 8 weeks after the first dose, and the third dose of the vaccine is administered to the subject at between 3 weeks and 26 weeks after the second dose. In certain embodiments, the vaccine is administered with an interval of about four weeks between the first and second administration, and an interval of about four weeks between the second and third administration.

In certain embodiments, the subject is a human subject. In certain embodiments, the human subject is between 6 months and 80 years of age, e.g. between 4 and 80 years of age. In certain embodiments, the subject is a subject that is not immunologically naive to influenza virus.

### Brief description of the Figures

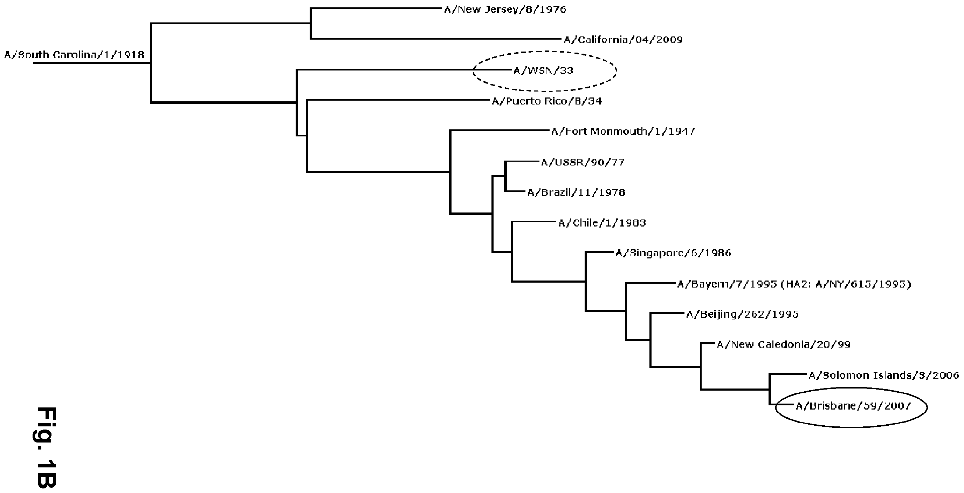

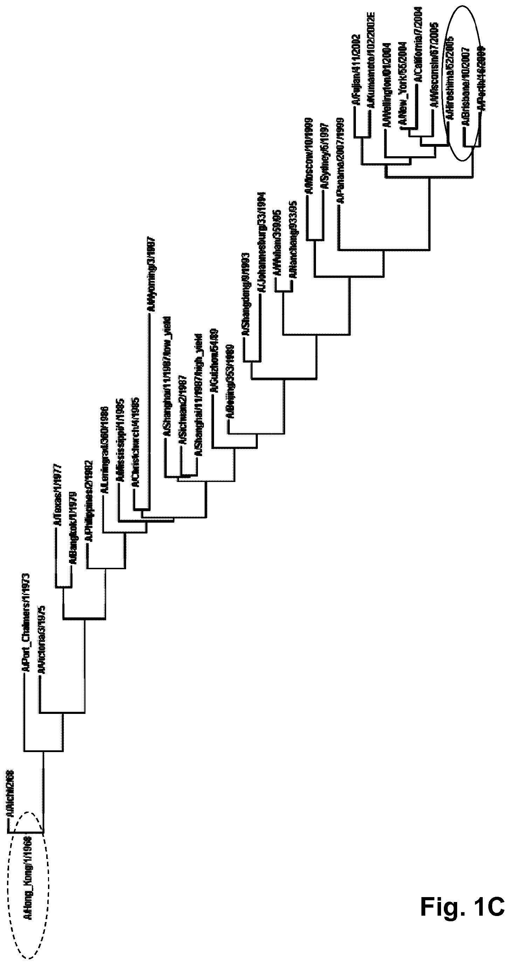

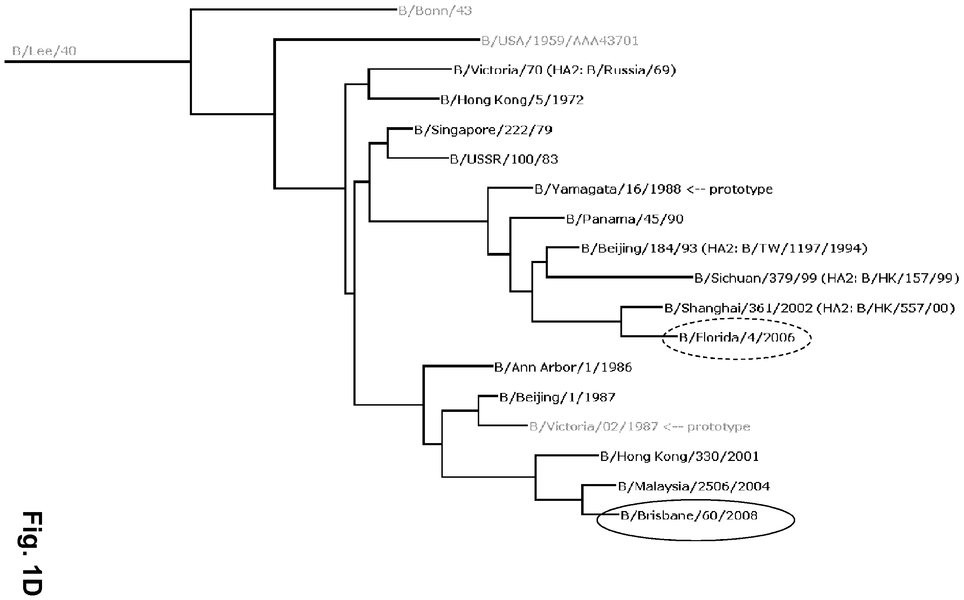

**Fig. 1****. A.** Phylogenetic tree of influenza A. **B.** Phylogenetic tree of H1N1 Influenza vaccine and selected strains. The vaccine strain used in example 1 is circled by a solid line, and the challenge strain used in example 1 is circled by a dotted line. **C.** Phylogenetic tree of H3N2 Influenza vaccine and selected strains. The vaccine strain used in example 3A is circled by a solid line, and the challenge strain used in example 3A is circled by a dotted line. **D.** Phylogenetic tree of Influenza B vaccine and selected strains. The vaccine strain used in example 4A is circled by a solid line, and the challenge strain used in example 4A is circled by a dotted line.

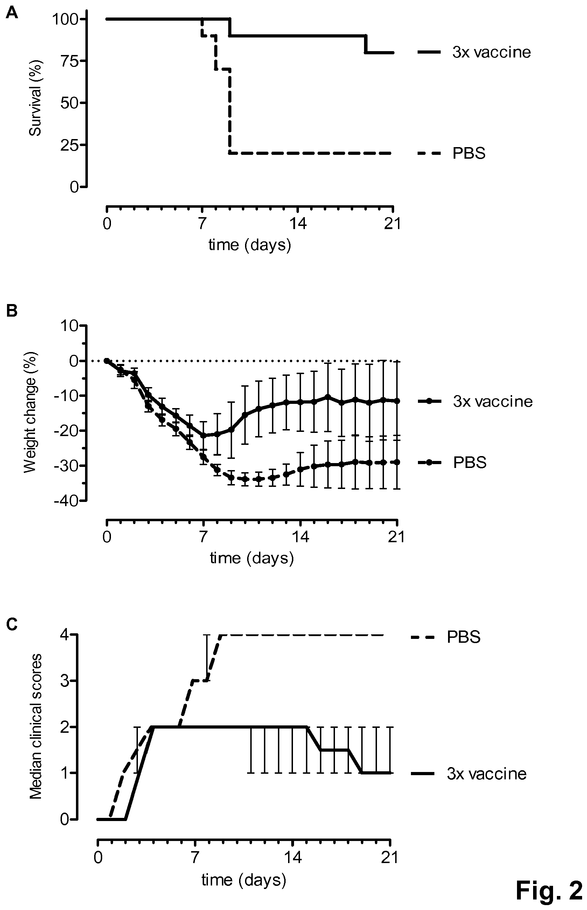

**Fig. 2****.** Results of experiment wherein mice where challenged with heterologous H1N1 influenza virus after 3x vaccination (solid line), or mock-vaccination with PBS (dashed line): **A.** survival, **B.** average relative body weight, **C.** clinical scores. For details see example 1.

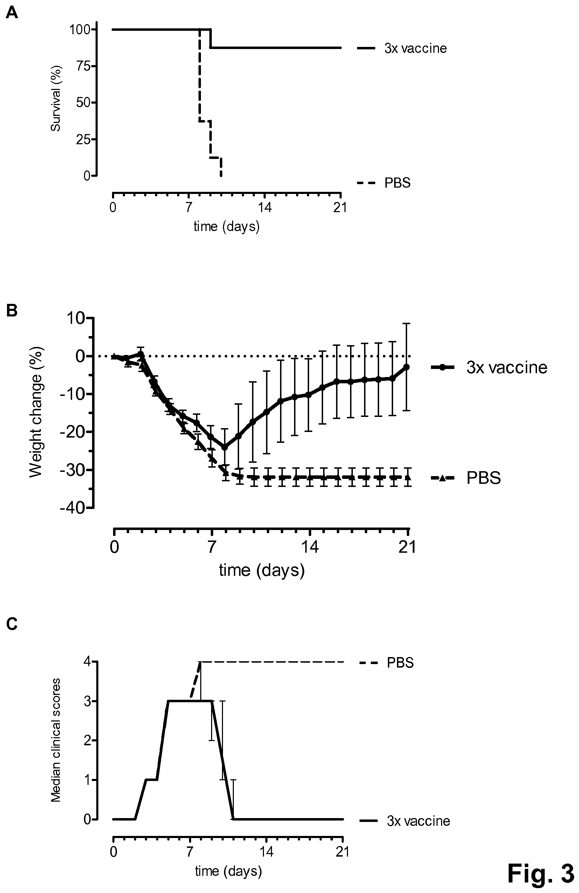

**Fig. 3****.** Results of experiment wherein mice where challenged with heterosubtypic H5N1 influenza virus after 3x vaccination (solid line), or mock-vaccination with PBS (dashed line): **A.** survival, **B.** average relative body weight, **C.** clinical scores. For details see example 2.

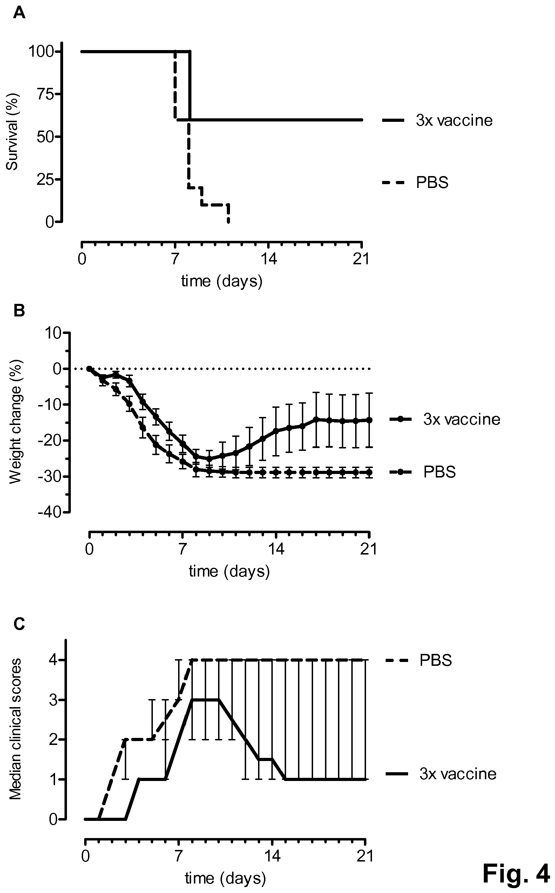

**Fig. 4****.** Results of experiment wherein mice where challenged with heterologous (H3N2) influenza virus after 3x vaccination (solid line), or mock-vaccination with PBS (dashed line): **A**. survival, **B.** average relative body weight, C. clinical scores. For details see example 3A.

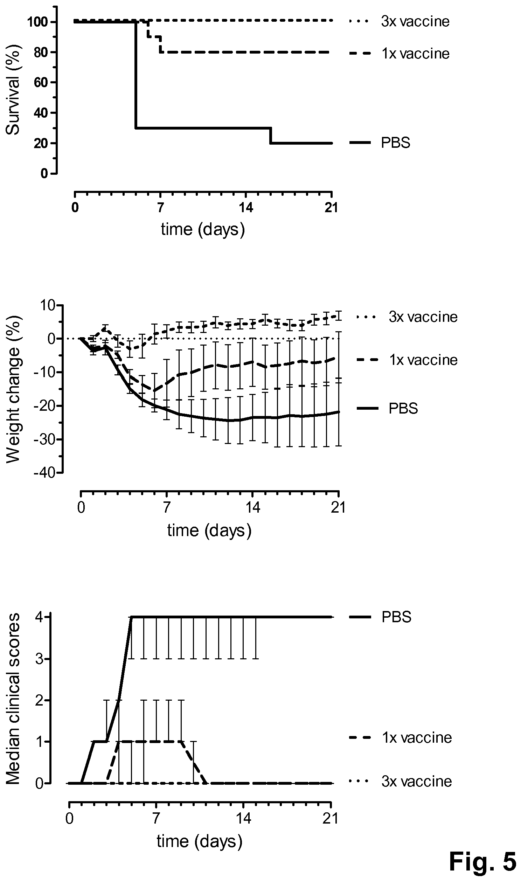

**Fig. 5****:** Results of experiment wherein mice were challenged with heterologous Influenza B/Florida/04/06 after 1x (dashed line) or 3x vaccination (speckled line) or mock vaccination with PBS (solid line). A. Survival, B average relative bodyweight, C clinical scores. For details see example 3B.

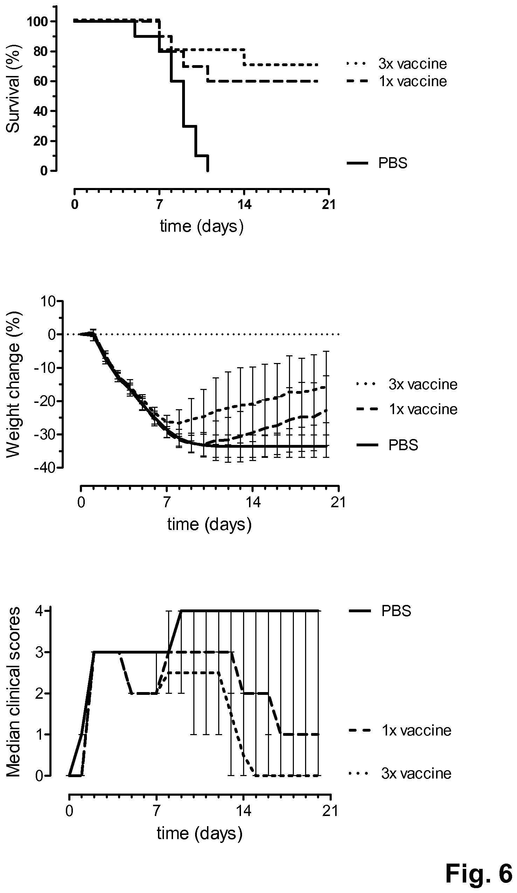

**Fig. 6****.** Results of experiment wherein mice were challenged with heterosubtypic H5N1 Influenza A/HK/156/97 after 1x (dashed line) or 3x vaccination (speckled line) or mock vaccination with PBS (solid line). A. Survival, B average relative bodyweight, C clinical scores. For details see example 3C.

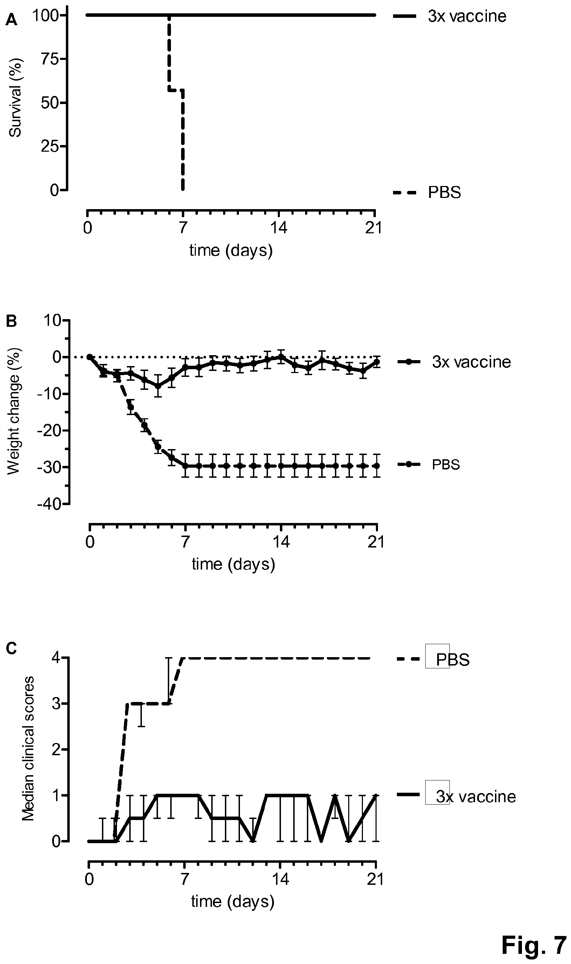

**Fig. 7****.** Results of experiment wherein mice where challenged with heterologous influenza B virus from the other clade after 3x vaccination (solid line), or mock-vaccination with PBS (dashed line): **A.** survival, **B.** average relative body weight, **C.** clinical scores. For details see example 4A.

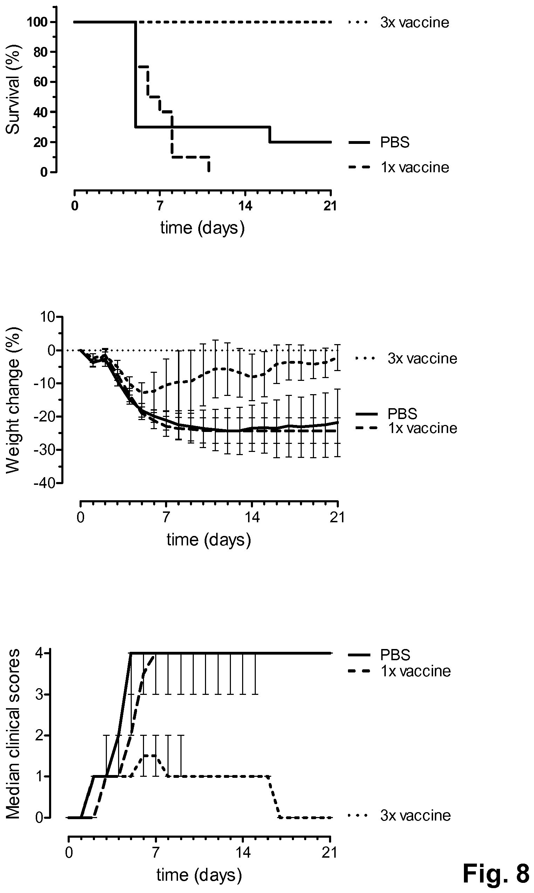

**Fig. 8****.** Results of experiment wherein mice were challenged with heterologous Influenza B/Florida/04/06 after 1x (dashed line) or 3x vaccination (speckled line) or mock vaccination with PBS (solid line). A. Survival, B average relative bodyweight, C clinical scores. For details see example 4B.

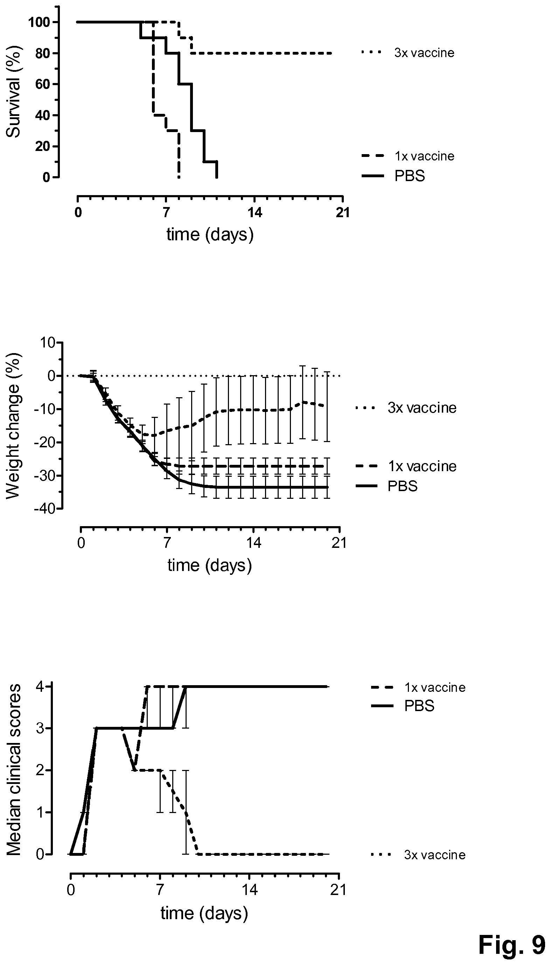

**Fig. 9****.** Results of experiment wherein mice were challenged with heterosubtypic H5N1 Influenza A/HK/156/97 after 1x (dashed line) or 3x vaccination (speckled line) or mock vaccination with PBS (solid line). A. Survival, B average relative bodyweight, C clinical scores. For details see example 4C

### Detailed description of the invention

An influenza strain is referred to herein as a 'heterologous' influenza strain compared to a first reference strain, when serum elicited by the strain no longer gives sufficient cross-neutralization of the reference strain (i.e. four-fold lower HI titer relative to homologous serum; e.g. Smith et al, 2004, Science 305: 371-376). Thus, e.g. when vaccine strains are updated, the new strain is by definition heterologous to the former strain.

A 'heterosubtypic' influenza strain as used herein is an influenza strain that compared to a first reference strain has a different HA subtype (e.g. H1N1 is heterosubtypic relative to H5N1 and to H3N2).

A 'seasonal' influenza strain is an influenza strain circulating in the human population at a certain moment. Such strains circulate during the influenza season, and can recur every year, usually in slightly altered form because of mutations in the antigenic regions (because of antigenic drift). A seasonal influenza vaccine has antigens from chosen seasonal influenza strains prevalent in that season. Typical examples of seasonal influenza strains at this moment are H1N1 strains, H3N2 strains, B strains.

A 'pandemic' influenza strain is a newly circulating influenza strain to which the population has little or no immunity. Non-limiting examples are H2N2, H5N1, H7N7 and H9N2 strains. However, for instance the 2009 Influenza pandemic was caused by an H1N1 strain, while other H1N1 strains were circulating.

According to the invention, the term 'protection' (also in the sense of 'cross-protection') to influenza strains, not necessarily implies protection against infection as such by these strains, but at least includes a reduction in the frequency, severity and/or duration of symptoms and/or complications that might result from infection by such influenza strains, when compared to a matching control group that has not been vaccinated. The term 'cross-protection' implies protection against at least one strain that is different (at least heterologous and preferably heterosubtypic) from the strains from which the HA and NA antigens are represented in the vaccine.

In certain embodiments, the HA and NA are from an influenza A strain. In certain embodiments thereof, the HA and NA are from an influenza A strain from phylogenetic group 1, such as from an influenza H1, H2, H5, H9, etc. subtype (see Fig. 1A for the subtypes in phylogenetic groups). In other embodiments, the HA and NA are from an influenza A strain from phylogenetic group 2, such as from an influenza H3, H4, H7, etc. subtype.

In other embodiments, the HA and NA are from an influenza B strain. For influenza B, two lineages, represented by the prototype viruses B/Victoria/2/87 (Victoria lineage) and B/Yamagata/16/88 (Yamagata lineage), are currently distinguished. B/Yamagata was the major lineage circulating until the 1980s, when B/Victoria lineage viruses appeared. Since then, drift variants of both influenza B lineages have been co-circulating globally, with both lineages concurrently circulating in recent influenza seasons. In certain embodiments, the vaccine of the invention comprises HA and NA from an influenza B strain, which can for instance be from the Yamagata-like lineage or from the Victoria-like lineage, or the vaccine may comprise HA and NA from B strains from both lineages.

The immunogenic proteins HA and NA, or immunologically active fragments thereof, may be formulated e.g. as split virus formulations, purified subunit formulations, viral proteins which are isolated, purified or derived from a virus, and virus like particles (VLPs). The influenza HA and NA proteins may also be obtained from other sources than from intact influenza virus, e.g. they may be obtained by recombinant expression in suitable expression systems, which are known to the skilled person. The vaccine may or may not comprise further influenza antigens besides HA and NA.

Depending on the particular season and on the nature of the antigen included in the vaccine, the influenza antigens may be derived from one or more of the following hemagglutinin subtypes: influenza A H1, H2, H3, H4, H5, H6, H7, H8, H9, H10, H11, H12, H13, H 14, H 15 or H16; or influenza B; and one or more of the following neuraminidase subtypes: influenza A N1, N2, N3, N4, N5, N6, N7, N8, or N9; or influenza B. For the majority of the HA subtypes, H1-H7 and H9- H12, all combinations with the 9 NA subtypes have been observed. Exemplary important combinations for influenza A comprise, but are not limited to: H1N1, H2N2, H3N2, H3N1, H5N1, H5N2, H7N7, H1N2, H9N2, H7N2, H7N3 and H10N7. Typically, a seasonal influenza vaccine comprises HA and NA of three influenza strains, which nowadays typically include a H1N1, a H3N2 and at least one B strain. Quadrivalent seasonal vaccines typically include antigens from an additional B strain (typically such vaccines comprise antigens from both a Yamagata and from a Victoria lineage B strain).

The influenza virus may also be a reassortant strain, and may have been obtained by reverse genetics techniques. Thus an influenza A virus may include one or more RNA segments from a A/PR/8/34 virus (typically 6 segments from A/PR/8/34, with the HA and N segments being from a vaccine strain, i.e. a 6:2 reassortant). It may also include one or more RNA segments from a A/WSN/33 virus, or from any other virus strain useful for generating reassortant viruses for vaccine preparation.

HA is the main immunogen in current inactivated influenza vaccines, and vaccine doses are standardized by reference to HA levels, typically measured by single radial immunodiffusion (SRID) (J. M. Wood et al., 1977, J. Biol. Stand. 5 (1977) 237-247). Existing vaccines typically contain about 15 µg of HA per strain, although lower doses can be used e.g. for children, or in pandemic situations, or when using an adjuvant. Fractional doses such as 1/2 (i.e. 7.5 µg HA per strain), 1/4 and 1/8 have been used, as have higher doses (e.g. 3x or 9x doses). Thus vaccines may include between 0.1 and 150 µg of HA per influenza strain, preferably between 0.1 and 50 µg eg. 0.1-20 g, etc. Particular doses include e g. about 15, about 10, about 7.5, about 5, about 1.5, etc µg per strain. Low doses can suitably be used in combination with intradermal administration.

The amount NA in a dose may vary, e.g. from about 0.1-50 µg, e.g. between about 0.5-20 µg. Typically, a seasonal vaccine may contain about 0.5-5 µg NA per 15 µg HA present, e.g. about 2-4 µg NA per 15 µg HA present.

HA and/or NA used with the invention may be a natural HA or NA as found in a virus, or may have been modified.

The vaccination regime according to the present invention is in principle suitable for all subjects. These include subjects of all ages, including for subjects that are and subjects that are not immunologically naive towards influenza virus at the moment of the administration of the first dose. A subject according to the invention preferably is a mammal that is capable of being infected with influenza, or otherwise can benefit from the induction of an immune response against influenza, such subject for instance being a rodent, e.g. a mouse, or a ferret, or domestic or farm animal, such as a pig, a dog, a horse, or a non-human-primate, or a human. Preferably, the subject is a human subject.

In certain embodiments, the subjects are human subjects having an age between about 6 months and 80 years old, e.g. between about 4 and 80 years old, e.g. between about 18 to 60 or 18 to 65 years old (adult population). In other embodiments, the subject is an elderly human subject (50 years or older, 60 years or older, 65 years or older). In other embodiments, a subject is a young human subject (e.g. ≤5 years old, e.g. between 6 months and 4 years old, i.e. a pediatric subject or a child). In other embodiments, subjects are hospitalized patients, healthcare workers, armed service and military personnel, pregnant women (in this embodiment the vaccine administrations provide cross protection to the mother but also could potentially induce cross-protection to the child after birth), the chronically ill (e.g. patients with asthma, diabetes, neurological and neuromuscular disorders (cerebral palsy, seizures, muscular dystrophy, etc)), immunodeficient patients, subjects who have taken an antiviral compound (e.g. an oseltamivir or zanamivir compound) in the 7 days prior to receiving the vaccine, etc. The vaccine may be used according to the invention generally in a population. In certain embodiments, the subject to which the vaccine is administered is not immunologically naive to an influenza virus antigen at the moment of the administration of the first dose. Generally in a population, human subjects before being 10 years old, e.g. of at least 4 years old, will have encountered influenza virus antigen either in nature or via vaccination. In other embodiments, the subject to which the vaccine is administered is immunologically naive to an influenza antigen virus at the moment of administration of the first dose.

In certain embodiments, the subject is a human pediatric subject, which is typically less than 3 years old, or less than 2 years old, or less than one year old. These can be immunologically naive to influenza virus, and typically a normal seasonal or pandemic influenza vaccine is already administered twice to such subjects, i.e. in a prime-boost regimen, in order to obtain sufficient immune response in such subjects. The methods of the present invention include administration of the vaccine at least three times, in order to broaden the immune response to obtain protection against influenza strains that are heterologous and even heterosubtypic to the strains from which the HA and NA in the vaccine are derived. This broadening of the immune response, by intramuscular, intradermal or subcutaneous administration and in the absence of adjuvant has not been reported before and goes against the dogma of strain-specific immunity that is induced by seasonal vaccination. Hence the finding of the broadened immune response upon triple administration in a prime-boost-boost regimen reported herein by the inventors was highly surprising.

In certain embodiments, the subject is a human subject of at least 3 months old, at least 6 months old, at least one year old, at least 2, 3, 4, 5, 6, 7, 8, 18, 50, 60, 65 years old. In certain embodiments, the subject is a human subject of not more than 50, 60, 65, 70, 80 years old. Any subject that would be eligible for regular seasonal and/or for a pandemic vaccination can also be vaccinated according to the invention.

For elderly or immunocompromised subjects, it may be desirable to include an adjuvant in the vaccine composition of at least one of the administrations, e.g. the first administration, e.g. all administrations. Independently or in addition, it may be beneficial to include a higher dose of the antigens in a vaccine that is administered to the elderly, e.g. two to four times higher antigen content as compared to a regular vaccine dose. A regular influenza vaccine dose generally contains about 15 µg of HA per strain.

A vaccine (also referred to as an "immunogenic composition") to be used according to the invention is preferably a pharmaceutical composition. It usually includes components in addition to the influenza antigens, e.g. it typically includes one or more pharmaceutically acceptable carrier(s) and/or excipient(s). The optimal ratios of each component in the formulation may be determined by techniques well known to those skilled in the art. In the present context, the term "pharmaceutically acceptable" means that the carrier or excipient, at the dosages and concentrations employed, will not cause unwanted or harmful effects in the subjects to which they are administered. Such pharmaceutically acceptable carriers and excipients are well known in the art (see *e.g.* Remington: The Science and Practice of Pharmacy (Gennaro, 2000; 20th edition, ISBN: 0683306472); Pharmaceutical Formulation Development of Peptides and Proteins, S. Frokjaer and L. Hovgaard, Eds., Taylor & Francis [2000]; and Handbook of Pharmaceutical Excipients, 3rd edition, A. Kibbe, Ed., Pharmaceutical Press [2000]). The vaccines preferably are formulated and administered as a sterile solution. Sterile solutions are prepared by sterile filtration or by other methods known per se in the art. The solutions can then be lyophilized or filled into pharmaceutical dosage containers. The pH of the solution generally is in the range of pH 3.0 to 9.5, e.g. pH 5.0 to 7.5. The HA and NA proteins or immunogenic parts thereof typically are in a solution having a suitable pharmaceutically acceptable buffer, and the solution may also contain a salt. In certain embodiments, detergent is present. In certain embodiments, the vaccine may be formulated into an injectable preparation. These formulations contain effective amounts of the antigens, are either sterile liquid solutions, liquid suspensions or lyophilized versions and optionally contain stabilizers or excipients. Compositions will generally be in aqueous form.

The composition may contain a physiological salt, for instance to control tonicity. NaCl is preferred, e.g. between 1-20 mg/ml, but also other salts may be present, such as potassium chloride, potassium dihydrogen phosphate, disodium phosphate dihydrate, magnesium chloride, calcium chloride, etc. In certain embodiments, lecithin may also be present. Generally the osmolality will be between 200-400 mOsm/kg. The composition may include one or more buffers, typically a phosphate buffer, a Tris buffer, a borate buffer, a succinate buffer, a histidine buffer, or a citrate buffer. Buffers will typically be included in the 5-20 mM range.

The composition is preferably non-pyrogenic. The composition may include one or more preservatives, such as thiomersal or 2-phenoxyethanol. Preferably, the vaccine is free of mercurial material such as thiomersal, more preferably free of preservative.

The composition may optionally include detergent e.g. a polyoxyethylene sorbitan ester surfactant (known as 'Tweens', e.g. Tween-20 or Tween-80), an octoxynol (such as octoxynol-9 (Triton X-100)), a cetyl trimethyl ammonium bromide ('CTAB'), sodium deoxycholate. The detergent may be present only at trace amounts.

Influenza vaccines are generally administered in a dosage volume of about 0.1 to 1.0 ml, typically about 0.5ml. For children, the administered dose is often half (i.e. about 0.25ml) of that used for adults.

Influenza vaccines are usually stored at between about 2°C and 8°C.

Various forms of influenza virus vaccine are currently available e.g. see chapters 17 & 18 of Plotkin & Orenstein (Vaccines, 4th edition, 2004, ISBN: 0-7216-9688-0). The ones used according to the invention are based on inactivated virus. Inactivated vaccines that can be used may be based on 'split' virions, or on purified surface antigens (including hemagglutinin). Influenza antigens can also be presented in the form of virosomes (nucleic acid free viral-like liposomal particles). Antigens purified from a recombinant host (e.g. in an insect cell line using a baculovirus vector, or in a mammalian cell line or in yeast cells) may also be used.

Typical influenza vaccines that may be used according to the invention are influenza vaccines that have been registered for seasonal vaccination, e.g. the ones marketed under the tradenames Inflexal® V (Crucell); Fluzone® or Vaxigrip® (Sanofi Pasteur); Fluvirin®, Agrippal®, Begrivac®, or Optaflu® (Novartis); Fluarix® (GSK); Imuvac® or Influvac® (Abbott); Fluvax® (CSL); Preflucel® (Baxter); etc.

A vaccine according to the invention may be any vaccine comprising HA and NA proteins, in the form of a virosomal vaccine. Several means for producing influenza vaccines are known and conventional to the skilled person.

The vaccine may be formulated in a sub-virion form e.g. in the form of a split virus, where the viral lipid envelope has been dissolved or disrupted, or in the form of one or more purified viral proteins. Methods of splitting viruses, such as influenza viruses, are well known in the art e.g. see WO02/28422, WO02/067983, WO02/074336, WO01/21151, etc. Splitting of the virus is carried out by disrupting or fragmenting whole virus, whether infectious (wild-type or attenuated) or non-infectious (e.g. inactivated), with a disrupting concentration of a splitting agent. Splitting agents generally include agents capable of breaking up and dissolving lipid membranes, typically with a hydrophobic tail attached to a hydrophilic head, e.g. non-ionic or ionic (e.g. cationic) surfactants, such as Triton, Tween, CTAB, etc. The disruption results in a full or partial solubilization of the virus proteins, altering the integrity of the virus.

Methods of purifying individual proteins from viruses are well known and include, for example, filtration, chromatography, centrifugation steps and hollow fiber elution. In one embodiment, the proteins are purified by ion exchange chromatography.

Methods of inactivating or killing influenza viruses to destroy their ability to infect mammalian cells are known in the art, and include for instance treatment with formalin, BPL, UV-light, etc.

The vaccine used in the invention is an influenza virosome vaccine. Thus, the influenza vaccine used in the methods of the present invention is a virosomal vaccine, which is a vaccine in which the majority, i.e. at least 50%, preferably at least 70%, of large lipid-protein complex structures is in the form of virosomes. Such virosomes comprise HA and NA proteins. In virosomal vaccines the virosomes are deliberately produced in the production process, e.g. by solubilisation of influenza particles using detergent, removal of insoluble materials, and subsequent defined removal of detergent to reconstitute the virosomes. In certain preferred embodiments the virosomes comprise exogenous lipids. Virosomes are reconstituted lipid membranes containing antigens from the virus. Virosomes can for instance be produced by two major approaches, one approach being based on the addition of exogeneous lipids during the reconstitution of the virosome (Almeida et al., 1975. Lancet 2:899-901; Trudel M. and F. Nadon, 1981. Can J Microbiol. 27:958-62; Ando et al., 1997. J Microencapsul. 14:79-90; Markgraf et al., 2001. Cloning 3:11-21; Gluck, R. and I. C. Metcalfe, 2002. Vaccine 20:B10-16; Mischler, R. and I. C. Metcalfe, 2002. Vaccine 20:B17-23) and the other being based on the formation of virosomes in the absence of addition of exogeneous lipids (Stegmann, T. et al. 1987. EMBO J. 6:2651-9; Huckriede et al., 2003. Vaccine 21:925-31). A particularly advantageous vaccine for use in the present invention is thus a virosomal influenza vaccine, such as Inflexal®V (see e.g. WO 92/19267; Glück, R, 1992, Vaccine 10: 915-920; Mischler, R. and I. C. Metcalfe, 2002. Vaccine 20:B17-23). These virosomes are also referred to as immunopotentiating reconstituted influenza virosomes (IRIVs). They mainly consist of spherical unilamellar vesicles with a diameter of between about 100 and 300 nm, e.g. approximately 150 nm, and the main constituents are naturally occurring phospholipids (PL) and phosphatidylcholine (PC), the latter forming about 70% of the virosomal structure, while the remaining 30% of membrane components are composed of envelope PLs originating from the influenza virus used to provide the HA and NA glycoproteins. They can be produced at large scale, from influenza virus that is solubilised by octaethyleneglycol (OEG), which after ultracentrifugation is mixed with lecithin. The process for preparing Inflexal® V is described in detail in the text and fig. 1 of Mischler, R. and I. C. Metcalfe, 2002. Vaccine 20:B17-23, incorporated by reference herein. Each dose (0.5 ml) of the Inflexal® V vaccine typically contains, as active component, 15 µg haemagglutinin of each of the influenza virus strains recommended seasonally by WHO, and additionally, each dose contains: 117 µg lecithin (phospolipid), 3.8 mg disodium hydrogen phosphate dehydrate, 0.7 mg potassium dihydrogen phosphate, 2.4 mg sodium chloride and 0.5 ml water for injection. The Inflexal® V virosomal influenza vaccine is commercially available from Crucell, and provides excellent immunogenicity without need for adjuvant, and low reactogenicity.

The HA and/or NA for the vaccines may be derived from influenza viruses that have been conventionally produced in fertilized hen's eggs. This well-known production method has been used for decades already, and still is the standard procedure for the vast majority of the influenza vaccines that are on the market.

Alternatively, the HA and NA may be derived from influenza viruses that were produced in suitable cell lines, such as mammalian, avian or insect cell lines, preferably mammalian cell lines such as MDCK, Vero, PER.C6, BHK, etc, using means and methods that as such are known and available to the skilled person (e.g described in EP 1951296). The use of mammalian cells means that vaccines can be free from materials such as chicken DNA, egg proteins (such as ovalbumin and ovomucoid), etc., thereby reducing allergenicity. WO97/37000, WO97/37001, EP1108787 and EP2290053 are examples where production of animal cells and cell lines that are capable of growth in suspension and in serum free media and are useful in the production and replication of influenza virus are described. Further details are given in WO03/023021 and WO03/023025. Cells may be grown in various ways e.g. in suspension, in adherent culture, on microcarriers. Production may preferably be in serum-free suspension culture.

The proteins of a subunit vaccine may also be produced by conventional recombinant DNA expression in suitable systems such as eukaryotic, preferably mammalian, cells.

Virions can be harvested from virus-containing fluids by various methods. For example, a purification process may involve zonal centrifugation using a linear sucrose gradient solution that includes detergent to disrupt the virions. Antigens may then be purified, after optional dilution, by diafiltration.

Purified surface antigen vaccines comprise the influenza surface antigens hemagglutinin and, typically, also neuraminidase. Processes for preparing these proteins in purified form are well known in the art.

The immune response raised by the methods of the invention will generally include an antibody response, preferably a protective antibody response. Methods for assessing antibody responses, neutralizing capability and protection after influenza vaccination are well known in the art. Human studies have shown that antibody titers against HA are correlated with protection (a serum sample hemagglutination-inhibition (HAI) titer of about 30-40 gives around 50% protection from infection by a homologous virus) [e.g. Potter & Oxford (1979) Br Med Bull 35: 69-75], although recent studies question this or even demonstrate a lack of correlation between HAI and efficacy.

Administration of the vaccine according to the invention can be performed using standard routes of parenteral administration, preferably by injection e.g. subcutaneous, intradermal, intramuscular. In one embodiment the vaccine is administered by intramuscular injection, e.g. into the deltoid muscle of the arm, or vastus lateralis muscle of the thigh. The skilled person knows the various possibilities to administer a vaccine according to the invention, in order to induce an immune response to the antigen(s) in the vaccine.

Multiple doses may be used in a prime-boost immunization schedule. In a typical embodiment, the at least three doses of the vaccine according to the invention are all given by intramuscular injection. In regimens of the invention a vaccine composition is comprising HA and NA from at least a first influenza strain, and the HA and NA proteins of the first influenza strain are administered at least three times within a period of less than one year. The administration schedule thus comprises a priming administratation and at least two boosting administrations. In the prime-boost schedule, preferably the same vaccine is used all three times, but it is also possible to make changes in certain components.

It has been described before to administer two doses of an influenza vaccine to immunologically naive subjects, but previously three doses of the same vaccine were not typically administered to the same subject, and the improved breadth of protection described for the first time herein upon three administrations of the influenza vaccine was highly surprising. Multiple doses will typically be administered according to the invention at least 1 week apart (e.g. about 2 weeks, about 3 weeks, about 4 weeks, about 6 weeks, about 8 weeks, about 10 weeks, about 12 weeks, about 16 weeks, etc.). The total time between first and third administrations according to the invention will be one year or less. Preferably, the total time between first and third administrations will be 10, 9, 8, 7, 6, 5 or 4 months or less. A month equals about 4 weeks, and these terms are used interchangeably herein. In certain embodiments, the second dose is administered between 1 and 12 weeks, preferably between 2 and 8 weeks, preferably between 3 and 6 weeks, after the first dose. In certain embodiments, the third dose is administered between 4 weeks and 10 months, preferably between 4 weeks and 6 months, after the second dose. In certain embodiments, the second dose is administered about 4 weeks after the first dose and the third dose is administered between about 4 weeks and 3 months, e.g. about 4 weeks, after the second dose. Such immunization schedules according to the invention have the advantage to ensure relatively quick onset of protection, and also induction of cross-protection to heterologous and even heterosubtypic influenza strains, with respect to all the strains from which the administered HA and NA antigens in the vaccine were derived.

Adjuvants are known in the art to further increase the immune response to an applied antigenic determinant, and pharmaceutical compositions comprising influenza antigens and suitable adjuvants are well known in the art, for instance the FluAd® influenza vaccine (Novartis) contains an adjuvant (MF59, an oil-in-water emulsion adjuvant comprising squalene). The terms "adjuvant" and "immune stimulant" are used interchangeably herein, and are defined as one or more substances that cause stimulation of the immune system. In this context, an adjuvant is used to enhance an immune response to the influenza antigens. The influenza vaccines used according to the invention do not comprise adjuvants. It was surprisingly found that adjuvants were not necessary to provide a broad immune response if a seasonal vaccine is administered three times to a subject, even by parenteral injection, since several prior art documents suggested that either mucosal administration or strong adjuvants or both were necessary to broaden the immune response against influenza vaccines. Some advantages of not including adjuvants in the vaccine are increased safety, lower reactogenicity, simpler product because less components need to be manufactured and tested, and potential use of the vaccines in young subjects.

The administration of the pharmaceutical compositions according to the inventions generally is aimed at inducing an immune response. The immune reponse may comprise a cellular and/or a humoral response. The cross-protection observed may comprise induction of antibodies but not necessarily against a conserved HA epitope.

The administration of the vaccine according to the methods of the invention induces cross protection against heterologous influenza strains and even heterosubtypic influenza strains. It may not provide sterile protection against such strains, i.e. may not protect against infection per se, but at least is aiming at reducing the frequency, severity and/or duration of at least one and preferably more or all of the symptoms from influenza infection by such strains, such as myalgia, headache, weakness/fatigue, cough, oropharyngeal pain, weight loss, chills, fever, muscle pain, coughing, general discomfort, etc. In addition, the frequency, severity and/or duration of complications that may occur from influenza infection by such strains (including for instance direct viral pneumonia or secondary bacterial pneumonia) which may even require hospitalization and can in severe cases even result in death, is expected to be reduced upon administering the vaccine according to methods of the invention. The methods of the invention may therewith also reduce or even prevent some of the unfavourable impact of influenza to the economy, e.g. absence from work, visits to doctor, hospitalization, requirement for (additional) medication, etc. The methods of the invention therefore have a great impact on health care. In addition, the methods of the invention allow vaccination against adverse consequences of infection with pandemic influenza strains by repeated use of standard seasonal vaccines, which can be a huge advantage in view of timing in view of earlier availability of vaccines during a pandemic outbreak.

The invention is further explained in the following examples. The examples do not limit the invention in any way. They merely serve to clarify the invention.

### EXAMPLES

### Example 1. Cross-protection to a heterologous influenza strain after 3 rounds of vaccination with a seasonal influenza vaccine in mice.

Mice were immunized with trivalent virosomal seasonal Influenza vaccine Inflexal® V (Crucell) of the 2009/2010 season. This vaccine contains HA (30µg/ml) and NA (amount not specified, generally determined to be between about 0.8 and 4 µg NA per 15 µg HA) of each of the following three Influenza strains: (H1N1 A/Brisbane/59/07, H3N2 A/Uruguay/716/2007, B/Brisbane/60/2008) in the form of virosomes. A human dose of vaccine is 15µg HA per strain, i.e. 0.5ml. Mice were vaccinated 3 times with 20% of the human dose at 3 week intervals (50 µl intramuscular injection both quadriceps muscles of the hind legs per immunization), using a normal injection syringe with needle. Four weeks after the final immunization mice were challenged with 25x LD50 (LD50 = dose of virus resulting in death of 50% of the exposed animals) of mouse adapted H1N1 Influenza strain WSN33, which is genetically distant from (and thus heterologous to) the vaccine H1 strain (see Fig. 1B). Mice that received 3x vaccine were compared to mice that received diluent injections (PBS, 2x50 µl intramuscular per injection) according to the same dosing schedule. After challenge body weight and clinical symptoms were monitored daily. Clinical scores were assigned as follows: 0 = no clinical signs; 1 = rough coat; 2 = rough coat, less reactive, passive during handling; 3 = rough coat, rolled up, labored breathing, passive during handling; 4 = rough coat, rolled up, labored breathing, unresponsive. Mice observed to have a clinical score of 4 were euthanized and scored as dead.

Fig. 2A shows the Kaplan-Meyer survival curve of the mice after challenge, and surprisingly shows that there is significantly increased survival (8 out of 10 mice immunized with 3x vaccine) relative to control mice (2 out of 10, p<0.023, using Fisher's exact test). As expected, 1 shot of vaccine did not confer protection in this model (not shown).

Fig. 2B shows average relative bodyweight of the mice after challenge, and shows that both groups of mice lose over 20% of weight, after which immunized mice mostly recover and un-immunized mice largely succumb to the infection. The last recorded bodyweight of mice prior to death or euthanization is carried forward in the analysis.

Fig. 2C shows clinical scores of the mice after challenge, and shows that immunized mice surviving the infection do not suffer from prolonged clinical illness. The last recorded clinical score prior to death or euthanization is carried forward in the analysis.

The composition of seasonal vaccines is modified almost annually when they are expected to no longer confer protection against genetically drifted circulating strains. Thus, it was highly unexpected that additional boosting with a 2009/2010 Influenza seasonal vaccine (i.e. three times administration of this same vaccine) confers protection against a genetically distant Influenza strain dating back to 1933. It is concluded that three times administration of the vaccine results in significant cross protection against a heterologous influenza strain.

### Example 2. Cross-protection to a heterosubtypic influenza strain after 3 rounds of vaccination with a seasonal influenza vaccine in mice.

Example 1 demonstrated cross protection against a heterologous H1N1 strain. In this example it was tested whether such cross protection would also be extended to heterosubtypic strains.

Mice were immunized according to the same dosing scheme and with the same vaccine as in example 1. Four weeks after the final immunization mice were challenged with 25x LD50 of mouse adapted H5N1 Influenza strain HK/156/97, which is heterosubtypic to the strains of which antigens are present in the vaccine. After challenge disease was monitored according to the same criteria as described for example 1.

Fig. 3A shows the Kaplan-Meyer survival curve of the mice after challenge, and surprisingly shows that there is significantly increased survival (7 out of 8 mice immunized with 3x vaccine) relative to control mice (0 out of 8, p<0.001, using Fisher's exact test).

Fig. 3B shows average relative bodyweight of the mice after challenge, and shows that both groups of mice lose over 20% of weight, after which immunized mice mostly recover and un-immunized mice rapidly succumb to the infection. The last recorded bodyweight of mice prior to death or euthanization is carried forward in the analysis.

Fig. 3C shows clinical scores of the mice after challenge, and shows that immunized mice surviving the infection do not suffer from prolonged clinical illness. The last recorded clinical score prior to death or euthanization is carried forward in the analysis.

Seasonal vaccines are composed of strains that are annually matched to circulating Influenza strains, and in general are not expected to provide protection against additional Influenza subtypes (see Fig. 1A for an overview). Specific vaccines are being developed for several Influenza subtypes that are suspected of posing a pandemic threat, such as H5N1. Thus, it was highly unexpected that additional boosting with a 2009/2010 Influenza seasonal vaccine confers protection against heterosubtypic H5N1 Influenza.

It is concluded that three times administration of the vaccine results in significant cross protection, not only against a heterologous influenza strain, but also against a heterosubtypic influenza strain.

### Example 3. Breadth of protection conferred by multiple administrations of Inflexal^{®} V 2009/2010 in mice.

The effect of seasonal influenza vaccine (H1N1 A/Brisbane/59/07, H3N2 A/Uruguay/716/2007, B/Brisbane/60/2008: Inflexal^{®} V,
season2009/2010) administered once, or three times (time interval between administrations: 3 weeks) is tested in mice, which are subsequently (4 weeks after final immunization) challenged with: i) heterologous H1, H3 and B strains, ii) a heterosubtypic H5 strain, and iii) a heterosubtypic H7 strain (methods as in example 1-3) in independent experiments.

It is expected that 3 administrations of the seasonal vaccine will significantly protect the animals against heterologous and heterosubtypic influenza strains, that are not represented (i.e. the antigens of these strains are not comprised) in the vaccine.

### Example 3A. Cross-protection to a heterologous H3 influenza strain after three rounds of vaccination with a seasonal influenza vaccine in mice.

Example 1 demonstrated cross protection against a heterologous H1N1 strain. In this example it was tested whether such cross protection would also be extended to heterologous H3 Influenza.

Mice were immunized according to the same dosing scheme and with the same vaccine as in example 1. Four weeks after the final immunization mice were challenged with 25x LD50 of mouse adapted H3N2 Influenza HK/1/68, which is strongly heterologous to the H3N2 strain that is present in the vaccine (see Fig. 1C). After challenge disease was monitored according to the same criteria as described for example 1.

Fig. 4A shows the Kaplan-Meyer survival curve of the mice after challenge, and shows that there is significantly increased survival (6 out of 10 mice immunized with 3x vaccine) relative to control mice (0 out of 10, p<0.05, using Fischer's exact test).

Fig. 4B shows average relative bodyweight of the mice after challenge, and shows that both groups of mice lose over 20% of weight, after which immunized mice mostly recover and un-immunized mice rapidly succumb to the infection. The last recorded bodyweight of mice prior to death or euthanization is carried forward in the analysis.

Fig. 4C shows clinical scores of the mice after challenge, and shows that immunized mice surviving the infection do not suffer from prolonged serious clinical illness. The last recorded clinical score prior to death or euthanization is carried forward in the analysis.

Seasonal vaccines are composed of strains that are annually matched to circulating Influenza strains, and in general only provide protection against the strains that are present in the vaccine and closely related strains. The challenge strain used in the current example is the original H3N2 pandemic strain from 1968, and is thus genetically distant from current H3N2 strains. Vaccine composition is updated based on much smaller degrees of genetic variation, which are expected to translate to reduced protection. Thus, prior to the present invention it was highly unexpected that seasonal Influenza vaccine given three times confers significant protection against genetically distant H3N2 Influenza. These results confirm that three times administration of seasonal influenza vaccine results in significant cross protection against a heterologous influenza strain, in this case for a H3 strain.

### Example 3B. Cross-protection to a heterologous Influenza B strain after one or three rounds of vaccination with a seasonal influenza vaccine in mice.

Example 1 demonstrated cross protection against a heterologous H1N1 strain. In this example it was tested whether such cross protection would also be extended to heterologous Influenza B for the 2009/2010 composition of seasonal vaccine. Mice were immunized with seasonal vaccine at 3 week intervals, and challenged 4 weeks after the final immunization with 25xLD50 of Influenza B/Florida/04/06. A control group receiving one shot of seasonal vaccine was immunized 4 weeks prior to challenge as well. After challenge disease was monitored according to the same criteria as described for example 1.

Fig. 5A shows the Kaplan-Meyer survival curve of the mice after challenge, and shows that 1x and 3x vaccine confer significant protection against the challenge compared to PBS control (survival 80%, p=0.01; 100%, p=0.001 respectively (2-sided Fischer's exact test).

Fig. 5B shows average relative bodyweight of the mice after challenge, and shows that both 1x and 3x vaccine significantly reduces bodyweight loss after challenge (p=0.032 and p<0.001 respectively, ANOVA with Dunnett post-hoc testing). In particular in the group receiving 3x vaccine weight loss after challenge is hardly observed at all.

Fig. 5C shows clinical scores of the mice after challenge, and shows that both 1x and 3x vaccine strongly reduce clinical symptoms relative to PBS control (significant from day 3 after challenge in both cases). Disease in treated groups is very mild, with median clinical score not exceeding 1.

Several companies have now moved to including 2 strains of Influenza B in a quadrivalent formulation of seasonal Influenza vaccine representing the 2 major clades of Influenza B (Victoria/02/1987) and Yamagata/16/1988 prototypes (Fig 1D)), based on the rationale that cross-protection is not expected between the 2 clades. Thus, prior to the present invention it was highly unexpected that mice immunized three times with a seasonal influenza vaccine in which only one clade of Influenza B is represented, are fully protected against heterologous Influenza B challenge. These results confirm that three times administration of a seasonal influenza vaccine results in significant cross protection against a heterologous influenza strain, in this case for a B strain. Moreover, the 2009 composition of vaccine already confers significant protection against heterologous Influenza B, while protection appears to be further improved by 3x seasonal vaccine.

### Example 3C. Cross-protection to heterosubtypic Influenza H5N1 after one or three rounds of vaccination with a seasonal influenza vaccine in mice.

Example 2 demonstrated cross protection against a heterosubtypic H5N1 strain. In the current example it is studied whether protection against heterosubtypic H5N1 Influenza is affected by the number of immunizations with vaccine (Inflexal® V, season 2009/10 composition). Mice were immunized with seasonal vaccine at 3 week intervals, and challenged 4 weeks after the final immunization with 25xLD50 of Influenza A/HK/156/97. A control group receiving one shot of seasonal vaccine was immunized 4 weeks prior to challenge as well. After challenge disease was monitored according to the same criteria as described for example 1.

Fig. 6A shows the Kaplan-Meyer survival curve of the mice after challenge, and shows that 1x and 3x vaccine confer significant protection against the challenge; survival 60%, p=0.022; 70%, p=0.006 respectively (2-sided Fischer's exact test).

Fig. 6B shows average relative bodyweight of the mice after challenge, and shows that 3x vaccine significantly reduces bodyweight loss (p= 0.023), ANOVA with Dunnett post-hoc testing). Based on bodyweight it is concluded protection by 3x vaccine is more robust, while percentages protection are comparable between 1x and 3x vaccine.

Fig. 6C shows clinical scores of the mice after challenge, and shows that both 1x and 3x vaccine immunized groups are substantially sick after challenge, however, both groups eventually recover, while controls succumb to the infection.

These findings confirm the findings in example 2. In addition, they identify that protection by 3x seasonal vaccine agains heterosubtypic H5N1 challenge appears to be more robust than protection by 1x seasonal vaccine.

### Example 4. Breadth of protection conferred by multiple administrations of Inflexal^{®} V 2010/2011 in mice.

The effect of seasonal influenza vaccine (H1N1 A/California/07/09, H3N2 A/Victoria/210/2009, B/Brisbane/60/2008: Inflexal® V, season
2010/2011) administered once, or three times (time interval between administrations: 3 weeks) is tested in mice, which are subsequently (4 weeks after final immunization) challenged with: i) homologous H1, H3 and B strains, ii) heterologous H1, H3, and B strains.

It is expected that 3 administrations of the seasonal vaccine will significantly protect the animals against heterologous and heterosubtypic influenza strains, that are not represented in the vaccine.

### Example 4A. Cross-protection to a heterologous Influenza B strain after three rounds of vaccination with a seasonal influenza vaccine in mice.

In this example it was tested whether 3x Immunization with a seasonal influenza vaccine (Inflexal® V, Season 2010/2011 composition) could confer protection against Influenza B (Flo/4/2006), belonging to the other clade of Influenza B as compared to the strain represented in the vaccine (see Fig. 1D).

Mice were immunized according to the same dosing scheme and with the same vaccine as in example 1. Four weeks after the final immunization mice were challenged with 25x LD50 of mouse adapted Influenza B Flordia/4/2006, which is strongly heterologous to the Influenza B strain that is present in the vaccine ((Brisbane/60/2008) see Fig. 1D). After challenge disease was monitored according to the same criteria as described for example 1.

Fig. 7A shows the Kaplan-Meyer survival curve of the mice after challenge, and surprisingly shows that there is significantly increased survival (10 out of 10 mice immunized with 3x vaccine) relative to control mice (0 out of 10, p<0.001, using Fischer's exact test).

Fig. 7B shows average relative bodyweight of the mice after challenge, and shows that the control group lose over 25% of their initial bodyweight before succumbing to the infection, whereas the 3x vaccine group lose less than 10% of their bodyweight (significant difference from day 3 onward). The last recorded bodyweight of mice prior to death or euthanization is carried forward in the analysis.

Fig. 7C shows clinical scores of the mice after challenge, and shows that 3x vaccine immunized mice surviving the infection do not suffer from serious clinical illness, whereas the control group rapidly progresses to serious clinical illness (as defined by a clinical score >2; difference significant from day 3). The last recorded clinical score prior to death or euthanization is carried forward in the analysis.

Seasonal vaccines are composed of strains that are annually matched to circulating Influenza strains, and in general only provide protection against the strains that are present in the vaccine and closely related strains. Several companies have now moved to including 2 strains of Influenza B in a quadrivalent formulation of seasonal Influenza vaccine representing the 2 major clades of Influenza B (Victoria/02/1987) and Yamagata/16/1988 prototypes (Fig 1D)), based on the rationale that cross-protection is not expected between the 2 clades. Thus, prior to the present invention it was highly unexpected that mice immunized three times with a seasonal influenza vaccine in which only one clade of Influenza B is represented, are fully protected against heterologous Influenza B challenge. These results confirm that three times administration of a seasonal influenza vaccine results in significant cross protection against a heterologous influenza strain, in this case for a B strain. Moreover, the results demonstrate that heterologous cross-protection by 3x vaccine is not confined to a single influenza vaccine composition, since it has been observed for both Inflexal 2009/2010 and 2010/11 compositions.

### Example 4B. Cross-protection to a heterologous Influenza B strain after one or three rounds of vaccination with a seasonal influenza vaccine in mice.

Example 4A demonstrated cross protection against a heterologous Influenza B for the 2010 composition of seasonal vaccine. In the current example we examined whether protection against heterologous Influenza B is affected by the number of immunizations with vaccine. Mice were immunized with seasonal vaccine (Inflexal® V, season 2010/11) at 3 week intervals, and challenged 4 weeks after the final immunization with 25xLD50 of Influenza B/Florida/04/06. A control group receiving one shot of seasonal vaccine was immunized 4 weeks prior to challenge as well. After challenge disease was monitored according to the same criteria as described for example 1.

Fig. 8A shows the Kaplan-Meyer survival curve of the mice after challenge, and shows that 3x vaccine confers complete protection against the challenge (100%, p=0.001, 2-sided Fischer's exact test). 1x vaccine does not significantly protect against heterologous Influenza B challenge.

Fig. 8B shows average relative bodyweight of the mice after challenge, and shows that only 3x vaccine significantly reduces bodyweight loss after challenge (p=0.018, ANOVA with Dunnett post-hoc testing).

Fig. 8C shows clinical scores of the mice after challenge, and shows that 3x vaccine strongly reduces clinical symptoms relative to PBS control (significant from day 3 after challenge), while 1x vaccine only causes a minor delay in symptoms. Disease in 3x vaccine group is very mild, with median clinical score not exceeding 1.5.

Similar results were obtained when another seasonal vaccine (Influvac®, a subunit vaccine of Abbot Healthcare, composition of season 2010/11 (i.e. same strains as in Inflexal® V for that season) was administered three times. While significant protection was already obtained after one administration, complete protection was only observed after 3 administrations.

These data clearly show an advantage of 3x immunization over 1x immunization with regard to protection against heterologous Influenza B.

### Example 4C. Cross-protection to heterosubtypic Influenza H5N1 after one or three rounds of vaccination with a seasonal influenza vaccine in mice.

Examples 2 and 3C demonstrated cross protection against a heterosubtypic H5N1 strain for the 2009 composition of seasonal Influenza vaccine. In the current example it is studied whether protection against heterosubtypic H5N1 Influenza is conferred by 1 or 3 immunizations with the 2010 composition of seasonal vaccine. Mice were immunized with seasonal vaccine (Inflexal® V, composition 2010/11) at 3 week intervals, and challenged 4 weeks after the final immunization with 25xLD50 of Influenza A/HK/156/97. A control group receiving one shot of seasonal vaccine was immunized 4 weeks prior to challenge as well. After challenge disease was monitored according to the same criteria as described for example 1.

Fig. 9A shows the Kaplan-Meyer survival curve of the mice after challenge, and shows that only 3x vaccine confers significant protection against the challenge (80%, p=0.001, 2-sided Fischer's exact test).

Fig. 9B shows average relative bodyweight of the mice after challenge, and shows that 3x vaccine significantly reduces bodyweight loss (p= 0.023), ANOVA with Dunnett post-hoc testing), while 1x vaccine does not significantly reduce bodyweight loss.

Fig. 9C shows clinical scores of the mice after challenge, and shows that both 1x and 3x vaccine immunized groups are substantially sick after challenge, however, only mice receiving 3x vaccine go on to recover (significant difference relative to PBS from day 7 onward).

These findings show that 3x seasonal vaccine of the 2010 composition also confers significant protection against heterosubtypic H5N1 challenge, extending the findings of example 2 and 3c. While 1x 2009 seasonal vaccine already conferred partial protection against H1N1 challenge, this was not the case for the 2010 vaccine. In both cases protection is clearly more robust after 3x immunization relative to 1x immunization.

### Example 5. Breadth of protection conferred by multiple administrations of influenza vaccine in ferrets.

Ferrets are considered a validated model for testing efficacy of influenza vaccines. The effect of seasonal influenza vaccine (H1N1, H3N2, B: Inflexal® V, season 2011/2012 or 2012/2013) administered once, twice or three times (time interval between administrations: 3 weeks) is tested in ferrets, which are subsequently (after 4 weeks) separately challenged with a heterosubtypic H5 strain (H5N1 Ind/05/05). Virus replication is assessed by culture of daily throat swabs, and survival is monitored up to 5 days after infection.

It is expected that 3 administrations of the seasonal vaccine will significantly protect the animals against heterosubtypic H5N1 Influenza.

### Example 6. Breadth of protection conferred by additional boost with alternative vaccines

The effect of alternative Influenza vaccines (e.g. alternative registered vaccine, inactivated whole Influenza virus, and/or recombinant HA + NA) administered once, twice or three times (time interval between administrations: 3 weeks) is tested in mice, which are subsequently (4 weeks after final immunization) challenged with: i) homologous H1, H3 and B strains, ii) heterologous H1, H3, and B strains, iii) heterosubtypic Influenza strains.

It is expected that additional boosting also with such vaccines will confer cross-protection against heterologous and heterosubtypic influenza strains, that are not represented in the vaccine.

### Example 7. Additional boosts with influenza vaccine V in human subjects

A clinical trial is performed with human subjects to test the safety and immunogenicity of seasonal influenza vaccine (H1N1 A/Brisbane/59/07, H3N2 A/Uruguay/716/2007, B/Brisbane/60/2008: Inflexal® V, season
2011/2012) administered three times (time interval between administrations: 4 weeks). Serum and peripheral blood mononuclear cells were collected prior to the start of every immunization, 4 weeks after the final immunization, and at 5 months after the primary immunization. Serum and peripheral blood mononuclear cells are collected after 12 months after the primary immunization.

Immunogenicity is monitored by homologous, heterologous and heterosubtypic neutralization, binding and HAI assays on collected serum. Immunogenicity data after one immunization showed that the vaccine met EMA criteria for all three strains. Cross-protection by serum is tested in mice, which receive an intraperitoneal injection with serum and are subsequently (1 day after injection) challenged with: i) homologous H1, H3 and B strains, ii) heterologous H1, H3, and B strains. iii) heterosubtypic Influenza strains.

## Claims

1. An influenza vaccine for use in inducing cross-protection against different influenza strains that include at least one heterologous strain as compared to the strains from which HA and NA are present in the influenza vaccine, by administering the influenza vaccine that comprises HA and NA from at least three influenza virus strains, wherein the vaccine is a virosomal influenza vaccine, and wherein the vaccine does not comprise an adjuvant and is administered intramuscularly to a subject at least three times within one year, and wherein no HA and NA from the at least one heterologous strain is administered to the subject.

2. An influenza vaccine for use in inducing cross-protection against at least one heterosubtypic influenza strain as compared to the influenza strains from which antigens are present in an influenza vaccine, by administering the influenza vaccine that comprises HA and NA, wherein the vaccine is a virosomal influenza vaccine, and wherein the vaccine does not comprise an adjuvant and is administered intramuscularly to a subject at least three times within one year.

3. A vaccine for use according to any one of the preceding claims, wherein the vaccine is administered with an interval between two administrations of at least one week, preferably between about three to eight weeks.

4. A vaccine for use according to any one of the preceding claims, wherein a second dose of the vaccine is administered to the subject at between 1 week and 8 weeks after a first dose, and a third dose of the vaccine is administered to the subject at between 3 weeks and 26 weeks after the second dose.

5. A vaccine for use according to any one of the preceding claims, wherein a second dose of vaccine is administered about one month after a first dose, and a third dose of vaccine is administered about one month after the second dose.

6. A vaccine for use according to any one of the preceding claims, wherein the vaccine comprises HA and NA from three or four influenza strains.

7. A vaccine for use according to claim 6, wherein the vaccine comprises HA and NA from a H1 strain, a H3 strain and a B strain.

8. A vaccine for use according to any one of the preceding claims, wherein the vaccine comprises HA and NA from an influenza H1N1 strain.

9. A vaccine for use according to any one of the preceding claims, wherein the subject is a human subject of between 6 months and 80 years of age.

10. A vaccine for use according to any one of claims 1-8, wherein the subject is a human subject of between 3 months and 4 years of age.

11. An influenza vaccine for use in inducing cross-protection against a H5N1 influenza strain, by administering an influenza vaccine that comprises HA and NA from an H1N1, an H3N2 and a B strain of influenza to a subject, wherein no HA and NA from the H5N1 influenza strain is administered to the subject, and wherein the vaccine is a virosomal influenza vaccine, and wherein the vaccine does not comprise an adjuvant and is administered intramuscularly at least three times within one year.

12. An influenza vaccine for use in vaccinating against influenza, the method comprising intramuscularly administering a virosomal influenza vaccine that does not comprise an adjuvant and that comprises influenza hemagglutinin (HA) and neuraminidase (NA) proteins from at least three influenza strains to a subject at least three times within one year.

13. A vaccine for use according to any one of the preceding claims, wherein the vaccine comprises HA and NA from at least an influenza H1N1 influenza strain, an H3N2 strain and a B strain.

14. A vaccine for use according to any one of the preceding claims, wherein the subject is immunologically naive to an influenza virus antigen at the moment of administration of the first dose of the vaccine.

15. An influenza vaccine comprising HA and NA proteins of at least three influenza strains, for use in inducing protection against the three influenza strains and cross-protection against at least one heterologous influenza strain within the same subtype as compared to at least one of the influenza strains, and also against at least one heterosubtypic influenza strain of which no HA and NA antigens are present in the vaccine, and wherein the vaccine is a virosomal influenza vaccine, and wherein the vaccine does not comprise an adjuvant and is administered intramuscularly to a subject at least three times within one year.

## Patentansprüche

1. Grippeimpfstoff zur Verwendung beim Induzieren eines Kreuzschutzes gegen unterschiedliche Influenzastämme, die wenigstens einen heterologen Stamm im Vergleich zu den Stämmen, aus denen HA und NA im Grippeimpfstoff vorliegen, umfassen, durch Verabreichen des Grippeimpfstoffs, der HA und NA aus wenigstens drei Influenzavirusstämmen umfasst, wobei es sich bei dem Impfstoff um einen virosomalen Grippeimpfstoff handelt und wobei der Impfstoff kein Adjuvans umfasst und einem Individuum wenigstens dreimal innerhalb eines Jahres intramuskulär verabreicht wird und wobei dem Individuum kein HA und NA aus dem wenigstens einen heterologen Stamm verabreicht wird.

2. Grippeimpfstoff zur Verwendung beim Induzieren eines Kreuzschutzes gegen wenigstens einen heterosubtypischen Influenzastamm im Vergleich zu den Influenzastämmen, aus denen Antigene in einem Grippeimpfstoff vorliegen, durch Verabreichen des Grippeimpfstoffs, der HA und NA umfasst, wobei es sich bei dem Impfstoff um einen virosomalen Grippeimpfstoff handelt und wobei der Impfstoff kein Adjuvans umfasst und einem Individuum wenigstens dreimal innerhalb eines Jahres intramuskulär verabreicht wird.

3. Impfstoff zur Verwendung nach einem der vorhergehenden Ansprüche, wobei der Impfstoff in einem Abstand von wenigstens einer Woche, vorzugsweise zwischen etwa drei bis acht Wochen, zwischen zwei Verabreichungen verabreicht wird.

4. Impfstoff zur Verwendung nach einem der vorhergehenden Ansprüche, wobei dem Individuum eine zweite Dosis des Impfstoffs zwischen 1 Woche und 8 Wochen nach einer ersten Dosis und eine dritte Dosis des Impfstoffs zwischen 3 Wochen und 26 Wochen nach der zweiten Dosis verabreicht werden.

5. Impfstoff zur Verwendung nach einem der vorhergehenden Ansprüche, wobei eine zweite Impfstoffdosis etwa einen Monat nach einer ersten Dosis und eine dritte Impfstoffdosis etwa einen Monat nach der zweiten Dosis verabreicht werden.

6. Impfstoff zur Verwendung nach einem der vorhergehenden Ansprüche, wobei der Impfstoff HA und NA aus drei oder vier Influenzastämmen umfasst.

7. Impfstoff zur Verwendung nach Anspruch 6, wobei der Impfstoff HA und NA aus einem H1-Stamm, einem H3-Stamm und einem B-Stamm umfasst.

8. Impfstoff zur Verwendung nach einem der vorhergehenden Ansprüche, wobei der Impfstoff HA und NA aus einem Influenza-H1N1-Stamm umfasst.

9. Impfstoff zur Verwendung nach einem der vorhergehenden Ansprüche, wobei es sich bei dem Individuum um ein menschliches Individuum im Alter zwischen 6 Monaten und 80 Jahren handelt.

10. Impfstoff zur Verwendung nach einem der Ansprüche 1-8, wobei es sich bei dem Individuum um ein menschliches Individuum im Alter zwischen 3 Monaten und 4 Jahren handelt.

11. Grippeimpfstoff zur Verwendung beim Induzieren eines Kreuzschutzes gegen einen H5N1-Influenzastamm durch Verabreichen eines Grippeimpfstoffs, der HA und NA aus einem H1N1-, einem H3N2- und einem B-Influenzastamm umfasst, an ein Individuum, wobei dem Individuum kein HA und NA aus dem H5N1-Influenzastamm verabreicht wird und wobei es sich bei dem Impfstoff um einen virosomalen Grippeimpfstoff handelt und wobei der Impfstoff kein Adjuvans umfasst und wenigstens dreimal innerhalb eines Jahres intramuskulär verabreicht wird.

12. Grippeimpfstoff zur Verwendung beim Impfen gegen Grippe, wobei das Verfahren intramuskuläres Verabreichen eines virosomalen Grippeimpfstoffs, der kein Adjuvans umfasst und der Influenza-Hämagglutinin(HA)- und -Neuraminidase(NA)-Proteine aus wenigstens drei Influenzastämmen umfasst, an ein Individuum wenigstens dreimal innerhalb eines Jahres umfasst.

13. Impfstoff zur Verwendung nach einem der vorhergehenden Ansprüche, wobei der Impfstoff HA und NA aus wenigstens einem Influenza-H1N1-Influenzastamm, einem H3N2-Stamm und einem B-Stamm umfasst.

14. Impfstoff zur Verwendung nach einem der vorhergehenden Ansprüche, wobei das Individuum zum Zeitpunkt der Verabreichung der ersten Dosis des Impfstoffs immunologisch naiv gegenüber einem Influenzavirusantigen ist.

15. Grippeimpfstoff, umfassend HA- und NA-Proteine von wenigstens drei Influenzastämmen, zur Verwendung beim Induzieren eines Schutzes gegen die drei Influenzastämme und eines Kreuzschutzes gegen wenigstens einen heterologen Influenzastamm innerhalb des gleichen Subtyps im Vergleich zu wenigstens einem der Influenzastämme und auch gegen wenigstens einen heterosubtypischen Influenzastamm, von dem keine HA- und NA-Antigene im Impfstoff vorliegen, und wobei es sich bei dem Impfstoff um einen virosomalen Grippeimpfstoff handelt und wobei der Impfstoff kein Adjuvans umfasst und einem Individuum wenigstens dreimal innerhalb eines Jahres intramuskulär verabreicht wird.

## Revendications

1. Vaccin contre la grippe à utiliser pour induire une protection croisée contre différentes souches de la grippe comprenant au moins une souche hétérologue comparativement aux souches à partir desquelles le HA et le NA sont présents dans le vaccin contre la grippe, en administrant le vaccin contre la grippe qui contient du HA et du NA à partir d'au moins trois souches du virus de la grippe, dans lequel le vaccin est un vaccin virosomal contre la grippe, et dans lequel le vaccin ne contient pas d'adjuvant et est administré de façon intramusculaire à un sujet au moins trois fois sur une année, et dans lequel aucun HA ni NA provenant de ladite au moins une souche hétérologue n'est administré au sujet.

2. Vaccin contre la grippe à utiliser pour induire une protection croisée contre au moins une souche de la grippe hétéro sous-typique comparativement aux souches de la grippe à partir desquelles des antigènes sont présents dans un vaccin contre la grippe, en administrant le vaccin contre la grippe qui contient du HA et du NA, dans lequel le vaccin est un vaccin virosomal contre la grippe, et dans lequel le vaccin ne contient pas d'adjuvant et est administré de façon intramusculaire à un sujet au moins trois fois sur une année.

3. Vaccin à utiliser selon l'une quelconque des revendications précédentes, dans lequel le vaccin est administré avec un intervalle entre deux administrations d'au moins un semaine, de préférence entre environ trois à huit semaines.

4. Vaccin à utiliser selon l'une quelconque des revendications précédentes, dans lequel une deuxième dose du vaccin est administrée au sujet entre 1 semaine et 8 semaines après une première dose, et une troisième dose du vaccin est administrée au sujet entre 3 semaines et 26 semaines après la deuxième dose.

5. Vaccin à utiliser selon l'une quelconque des revendications précédentes, dans lequel une deuxième dose de vaccin est administrée environ un mois après une première dose, et une troisième dose de vaccin est administrée environ un mois après la deuxième dose.

6. Vaccin à utiliser selon l'une quelconque des revendications précédentes, dans lequel le vaccin contient du HA et du NA provenant de trois ou quatre souches de la grippe.

7. Vaccin contre la grippe à utiliser selon la revendication 6, dans lequel le vaccin contient du HA et du NA provenant d'une souche H1, d'une souche H3 souche et d'une souche B.

8. Vaccin à utiliser selon l'une quelconque des revendications précédentes, dans lequel le vaccin contient du HA et du NA provenant d'une souche H1N1 de la grippe.

9. Vaccin à utiliser selon l'une quelconque des revendications précédentes, dans lequel le sujet est un sujet humain dont l'âge est compris entre 6 mois et 80 ans.

10. Vaccin à utiliser selon l'une quelconque des revendications 1 à 8, dans lequel le sujet est un sujet humain dont l'âge est compris entre 3 mois et 4 ans.

11. Vaccin contre la grippe à utiliser pour induire une protection croisée contre une souche de la grippe H5N1, en administrant un vaccin contre la grippe qui contient du HA et du NA provenant d'une souche H1N1, H3N2 et B de la grippe à un sujet, dans lequel aucun HA ni NA provenant de la souche H5N1 de la grippe n'est administré au sujet, et dans lequel le vaccin est un vaccin virosomal contre la grippe, et dans lequel le vaccin ne contient pas d'adjuvant et est administré de façon intramusculaire au moins trois fois sur une année.

12. Vaccin contre la grippe à utiliser pour la vaccination contre la grippe, le procédé comprenant l'administration intramusculaire d'un vaccin virosomal contre la grippe qui ne contient pas d'adjuvant et qui contient des protéines d'hémagglutinine (HA) et de neuraminidase (NA) de la grippe provenant d'au moins trois souches de la grippe à un sujet au moins trois fois sur une année.

13. Vaccin à utiliser selon l'une quelconque des revendications précédentes, dans lequel le vaccin contient du HA et du NA provenant d'au moins une souche H1N1, une souche H3N2 et une souche B de la grippe.

14. Vaccin à utiliser selon l'une quelconque des revendications précédentes, dans lequel le sujet est immunologiquement exempt de tout antigène du virus de la grippe au moment de l'administration de la première dose du vaccin.

15. Vaccin contre la grippe contenant des protéines de HA et de NA d'au moins trois souches de la grippe, à utiliser pour induire une protection contre les trois souches de la grippe et une protection croisée contre au moins une souche hétérologue de la grippe à l'intérieur du même sous-type comparativement à au moins une des souches de la grippe, et également contre au moins une souche hétéro sous-typique de la grippe dont aucun antigène de HA et de NA n'est présent dans le vaccin, et dans lequel le vaccin est un vaccin virosomal contre la grippe, et dans lequel le vaccin ne contient pas d'adjuvant et est administré de façon intramusculaire à un sujet au moins trois fois sur une année.
